# FASCICULE DE BREVET EUROPEEN

(11) **EP 2 088 934 B1**
(45) Date de publication et mention de la délivrance du brevet: **20.06.2012**
(21) Numéro de dépôt: 07870254.5
(22) Date de dépôt: 07.11.2007
(51) Int. Cl.: A61B 17/02, A61B 17/00

(54) **ECARTEUR LAPAROSCOPIQUE.**
LAPAROSKOPISCHES DISTANZSTÜCK
LAPAROSCOPIC SPACER

(30) Priorité: 07.11.2006 FR 0609723
(43) Date de publication de la demande: 19.08.2009
(73) Titulaire: Corpataux, Jean-Marc, 1010 Lausanne (CH); Cau, Jérôme, 86000 Poitiers (FR)
(72) Inventeur: CAU, Jérôme, 86000 Poitiers (FR)
(74) Mandataire: L'Helgoualch, Jean
(86) Numéro de dépôt international: PCT/FR2007/001838
(87) Numéro de publication internationale: WO 2008/068405

(56) Documents cités:
- EP-A2- 0 531 710
- WO-A-95/07052
- WO-A-03/094744
- US-A- 5 235 966

## Description

L'invention concerne un écarteur laparoscopique destiné à maintenir à distance un premier organe d'un deuxième organe, lors d'une intervention chirurgicale laparoscopique.

De telles interventions nécessitent l'utilisation de trocarts optiques et de trocarts opérateurs par l'intermédiaire desquels différents instruments chirurgicaux sont introduits dans la cavité transpéritonéale, qui a été préalablement insufflée sous pression constante.

Lors de ces interventions, l'une des difficultés majeures est de maintenir une position stable des différents organes, qui soit non seulement susceptible de faciliter le travail du chirurgien en exposant convenablement les organes à manipuler, mais qui évite également toute gêne, notamment respiratoire, chez le patient au cours de l'intervention.

La position des anses intestinales est particulièrement concernée, dans la mesure où celles-ci sont relativement volumineuses et mobiles. Par exemple, dans le cadre d'interventions du type restauration aortique abdominale, les anses intestinales empêchent un accès aisé à l'aorte, et constituent un obstacle au champ de vision du chirurgien.

Pour contourner cette difficulté, différentes approches ont été proposées. L'une de ces approches est d'utiliser un dispositif de type écarteur, qui permet d'écarter et de maintenir en position les viscères susceptibles de gêner l'intervention.

Le document WO 06/069947 décrit un écarteur-rétenteur d'intestins pour chirurgie coelioscopique, qui est constitué de plusieurs éléments devant être insérés les uns dans les autres par le chirurgien au cours de l'intervention afin de s'adapter à l'anatomie du patient. Un tel dispositif est de maniement complexe pour le chirurgien. En outre, cet écarteur ne peut pas être utilisé lors d'interventions dites totalement laparoscopiques, dans lesquelles l'intervention dans son ensemble est réalisée par voie coelioscopique, y compris le temps de restauration chirurgicale. Il est en effet destiné à des procédures chirurgicales par voie mini invasive ou à des procédures mixtes dans lesquelles, après une première étape de dissection sous coelioscopie, le geste de restauration proprement dit est réalisé par l'intermédiaire d'une incision abdominale sans coelioscopie, comme en chirurgie conventionnelle.

On connaît également, de US 5.178.133, un écarteur laparoscopique comprenant deux bras au milieu desquels s'étend une membrane élastique destinée à retenir les organes ou tissus concernés, les deux bras pouvant être ouverts ou fermés à la manière de ciseaux afin d'ajuster leur écartement. Cet écarteur présente l'inconvénient de ne pas pouvoir être utilisé pour des restaurations complexes, telles que celle portant sur l'aorte située en position profonde dans l'abdomen, contre le rachis dorso-lombaire, et qui est par conséquent difficile d'accès. L'utilisation de cet écarteur est donc limitée à la manipulation d'organes intra abdominaux directement accessibles, tels que le foie. En outre, du fait de sa rigidité, un tel écarteur est susceptible de créer des lésions des organes pleins, lors de sa manipulation au sein de la cavité abdominale. Un autre écarteur de ce type est décrit dans la demande WO 95/07052 et comprend deux lames rigides portant un filet souple, pouvant pivoter et s'écarter angulairement l'une de l'autre suivant un angle contrôlé par un fil rigide reliant la base des lames à un coulisseau qui peut être manoeuvré à partir de la poignée de l'écarteur. La demande WO 03/094744, sur laquelle est basé le préambule de la revendication 1, décrit un écarteur comprenant un axe agissant sur plusieurs lames rigides mobiles par pivotement et reliées entre elles par un filet.

Le document US 5.755.661 décrit un écarteur constitué de plusieurs bras susceptibles de se déployer en formant une surface plane. Cet écarteur est plus particulièrement destiné à agrandir la paroi abdominale lors d'une intervention, et ne permet donc pas d'écarter un organe spécifique afin de faciliter l'accès à l'organe ou au tissu devant être manipulé par le chirurgien.

Les écarteurs connus de l'état de la technique ne sont donc pas pleinement satisfaisants car ils sont, pour certains, de manipulation complexe, ce qui implique une précision du geste plus difficile à acquérir par le chirurgien et des durées d'intervention plus longues. En outre, certains d'entre eux ne peuvent pas être utilisés lors de procédures réalisées entièrement par voie laparoscopique, et ils peuvent également créer des lésions sur les organes qui les entourent.

La publication de J. Cau et al., Journal of Vascular Surgery, Vol. 41 n°5 (2005), pages 902-906, a pour objet un écarteur comprenant deux lames disposées dans une gaine, et. entre lesquelles est prévu un filet de rétention destiné à envelopper et retenir les anses intestinales. Les lames sont aptes à s'ouvrir lorsqu'elles sont poussées hors de la gaine, ce qui conduit au déploiement du filet dans la cavité abdominale.

L'invention propose un écarteur agencé pour permettre le déploiement automatique des lames et l'ajustement précis de leur écartement. Cet écarteur présente l'avantage d'être simple à manipuler, utilisable dans divers types d'interventions, facilement adaptable à la morphologie de chaque patient, et il autorise des gestes précis et rapides de l'utilisateur.

A cet effet, l'invention a pour objet un écarteur laparoscopique selon la revendication 1 destiné à maintenir à distance un premier organe d'un deuxième organe lors d'une opération chirurgicale laparoscopique pratiquée sur ledit deuxième organe.

Ledit écarteur comprend un dispositif de rappel agencé pour ajuster et maintenir l'écartement entre les lames, ledit dispositif comprenant des éléments de rappel constitués d'au moins une lamelle souple à deux branches formant sensiblement un V, disposée entre lesdites lames, chaque extrémité des branches du V étant reliée respectivement à chacune des deux lames au voisinage de l'extrémité distale dudit tube, et des moyens de commande des éléments de rappel, lesdits moyens de commande étant disposés sur la poignée.

Un tel dispositif présente l'avantage de se déployer automatiquement une fois placé à l'endroit souhaité, de sorte à constituer une barrière et empêcher l'envahissement du champ opératoire par les viscères. Il assure ainsi une exposition stable dans le temps de l'organe à opérer, sans autre manipulation, et sans nécessiter d'incision abdominale. En outre, le dispositif de rappel prévu autorise l'ajustement simple et précis de l'écartement des lames aux dimensions de l'organe qu'il est nécessaire de retenir.

Le fait que l'écarteur selon l'invention puisse se déployer automatiquement et puisse être ajusté précisément limite en outre les manipulations à effectuer par le chirurgien, ce qui diminue le risque de lésions sur les organes ou les tissus environnants. Cette particularité rend possible l'utilisation de l'écarteur de l'invention suivant la technique "NOTES" (Natural Orifice Transluminal Endoscopic Surgery). Ainsi l'intervention chirurgical est réalisée sans aucune incision cutanée, à l'exception de l'introduction d'une aiguille de 2 mm permettant l'insufflation et le contrôle de la pression intra-abdominale, les instruments étant notamment introduits soit par la voie transgastrique, soit trans-vaginale ou trans-rectale. Cette technique chirurgicale présente l'avantage de permettre une diminution, voire l'absence de douleur postopératoire, de faciliter l'accès à certains organes, d'éviter tout traumatisme de la paroi abdominale ; elle présente en outre un avantage cosmétique dans la mesure où elle évite toute cicatrice.

Selon une réalisation, les éléments de rappel sont constitués d'au moins une lamelle souple à deux branches formant sensiblement un V, chaque extrémité des branches du V étant reliée respectivement à chacune des deux lames, et d'une tige filetée pénétrant au moins partiellement dans ledit tube, ladite tige filetée comprenant une extrémité distale apte à s'étendre à l'extérieur du tube lorsque les lames sont dans leur deuxième position, et venant en butée contre la au moins une lamelle dans le V formé par les deux branches. Selon cette réalisation, les moyens de commande des éléments de rappel sont constitués d'une molette coopérant avec la tige filetée.

Les lames préformées sont de préférence formées d'un métal souple, de type acier ou matière plastique injectée.

Lorsqu'on utilise un filet entre les deux lames, celui-ci est formé par exemple d'une maille de polypropylène.

L'écartement automatique et atraumatique des lames de l'écarteur est rendu possible par la forme particulière des lames et du filet.

A cet effet chacune des lames, lorsqu'elles sont libres et qu'elles ne sont pas reliées par le filet, a une forme en "S". L'armature du filet est constituée par les deux lames distinctes rattachées à l'extrémité distale du manche, par leur extrémité proximale.

L'angle formé par le manche et la droite reliant les extrémités distale et proximale de chaque lame, avant mise en place du filet, est compris de préférence entre 90° et 110°.

De plus, la forme initiale en "S" de chaque lame permet de donner une forme concave au filet une fois déployé, rendant le filet plus efficace pour récliner un organe tel que l'intestin grêle. Cette forme présente en outre l'avantage d'être plus anatomique et moins traumatisante.

Lorsque le filet est mis en place sur les lames, sa configuration maintient chaque lame dans une position angulaire de 30° environ par rapport à l'axe du manche. Cette différence angulaire entre les positions des lames avant et après mise en place du filet est à l'origine du déploiement harmonieux et atraumatique de l'écarteur.

La force de déploiement du filet permet son expansion sans autre manipulation externe, jusqu'à sa forme finale, sans qu'il soit nécessaire d'utiliser d'autres instruments introduits dans la cavité abdominale par des trocarts, comme dans les écarteurs connus, ce impose de multiplier les incisions. L'écarteur de l'invention permet donc d'éviter cet inconvénient.

De plus pour se déployer, l'écarteur de l'invention ne nécessite pas non plus un mécanisme de réglage, comme une vis montée rotative par rapport au manche, ce qui réduit le risque de blesser des organes.

Le dispositif de rappel permet simplement de rigidifier l'écarteur une fois déployé et positionné.

Suivant une variante, les deux lames peut être réunies pour former une lame unique fixée au manche de l'écarteur par son milieu.

L'invention a également pour objet un dispositif laparoscopique comprenant un écarteur tel que défini précédemment et un trocart endoscopique, ledit écarteur étant placé à l'intérieur dudit trocart.

L'écarteur laparoscopique de l'invention peut être fourni au chirurgien avec le filet en place sur les lames, ou avec le filet non monté, c'est-à-dire en laissant les lames libres, et dans ce cas le chirurgien doit mettre en place le filet sur les lames au moment de son intervention.

D'autres objets et avantages de l'invention apparaîtront au cours de la description qui suit, faite en référence aux dessins annexés dans lesquels :
- les figures la et 1b sont respectivement une vue en perspective et une vue en coupe longitudinale d'un écarteur selon l'invention, dans lequel les lames sont disposées à l'intérieur de la gaine, dans leur première position ;
- la figure 2 est une vue en perspective d'un écarteur selon l'invention dans lequel la gaine a coulissé sur le tube rigide, les lames étant en train de passer dans leur deuxième position;
- les figures 3a et 3b sont respectivement une vue de côté et une vue arrière d'un écarteur qui ne comprend pas de filet ou de membrane, montrant l'écartement maximal que pourraient atteindre les lames lors de leur déploiement automatique;
- la figure 4 est une vue en perspective d'un écarteur selon l'invention dans lequel les lames sont dans leur deuxième position, l'écartement entre les lames ayant été ajusté à l'aide du dispositif de rappel.

L'écarteur 1 représenté sur les figures est plus particulièrement destiné à écarter les anses intestinales lors d'une intervention laparoscopique consistant en une restauration aortique abdominale.

Pour cette utilisation, l'écarteur 1 est placé à l'intérieur d'un trocart endoscopique (non représenté) dont le diamètre est environ égal à 10 mm.

L'écarteur 1 comprend une poignée 2 sensiblement cylindrique qui est équipée de moyens (non représentés) permettant de la fixer à une table d'opération.

La poignée 2 est prolongée par un tube creux rigide 3 dont l'extrémité proximale 4 est reliée à la poignée 2, et dont l'extrémité distale 5 est prolongée par deux lames flexibles 6a, 6b.

Par les termes "proximal" et "distal", on entend les parties du dispositif situées respectivement à proximité de l'utilisateur et à distance de celui-ci.

Avant d'être montées sur l'écarteur 1, les deux lames 6a, 6b ont été préformées sensiblement en forme de S, de sorte à pouvoir ultérieurement se déployer selon une configuration précise.

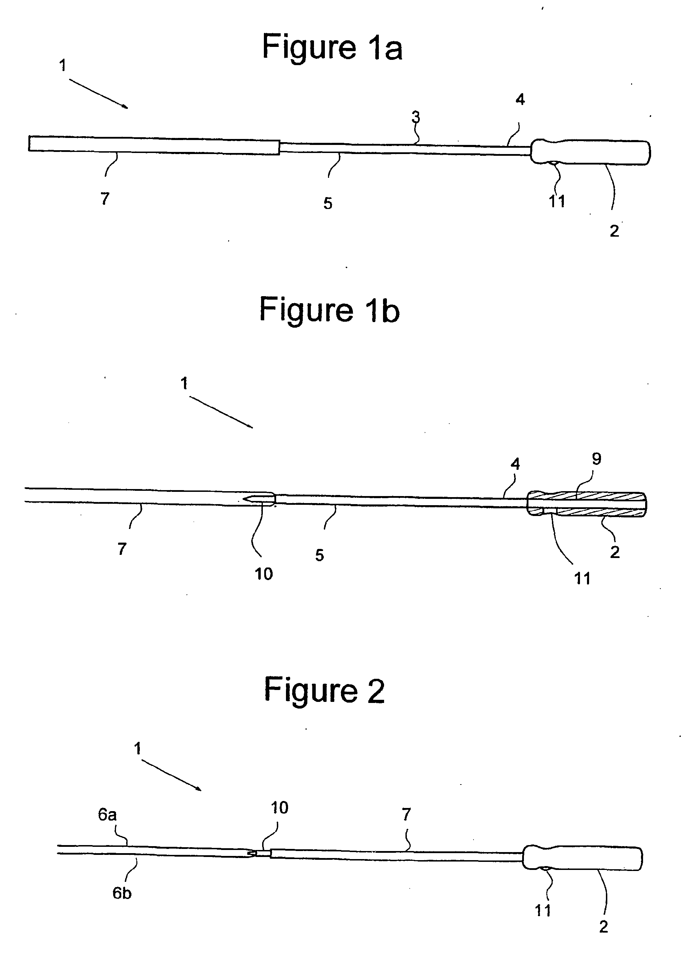

L'une des extrémités de chaque lame 6a, 6b est fixée à l'extrémité distale 5 du tube rigide 3, de telle sorte que les lames 6a, 6b, dans une première position, soient sensiblement parallèles l'une à l'autre (voir figure 1b).

L'écarteur 1 comprend également un filet souple (non représenté) disposé entre les deux lames 6a, 6b, et fixé à celles-ci par l'intermédiaire de ses bordures.

Ce filet est prévu de dimensions suffisantes pour permettre l'enveloppement au moins partiel des anses abdominales. Ses dimensions sont prévues selon la taille ou le volume de la cavité abdominale du patient. Pour une petite cavité dont le volume est inférieur à 3 litres après insufflation du pneumopéritoine, les dimensions du filet sont de 10 cm de largeur sur 15 cm de longueur une fois déployé, la largeur correspondant à la dimension du filet entre les deux lames déployées. Pour une cavité considérée comme normale, dont le volume est compris entre 3 et 4,5 litres après insufflation du pneumopéritoine, les dimensions du filet sont de 15 cm de largeur sur 20 cm de longueur une fois déployé, et pour une cavité abdominale très volumineuse, dont le volume est supérieur à 4,5 litres après insufflation du pneumopéritoine, les dimensions du filet sont de 15 cm de largeur sur 25 cm de longueur une fois déployé. Les dimensions des lames sont adaptées à celles du filet, leur épaisseur peut être d'environ 1 mm et leur largeur d'environ 3 mm.

Une gaine 7 sensiblement cylindrique est prévue sur l'écarteur 1 afin de recevoir les lames 6a, 6b dans leur première position (figures 1a et 1b). A l'intérieur de la gaine 7, les lames 6a, 6b sont en contact l'une avec l'autre par l'intermédiaire de leurs bords convexes.

Les dimensions de la gaine 7 sont agencées pour lui permettre non seulement de recevoir entièrement les deux lames 6a, 6b en position sensiblement parallèle, mais également, comme cela apparaît sur la figure 2, pour pouvoir coulisser au-dessus du tube rigide 3 de sorte à libérer les lames 6a, 6b et permettre leur passage dans une deuxième position.

L'écarteur 1 est en outre muni d'un dispositif de rappel permettant d'ajuster et de maintenir l'écartement entre les lames 6a, 6b, lorsque celles-ci sont placées dans leur deuxième position, en se déployant automatiquement.

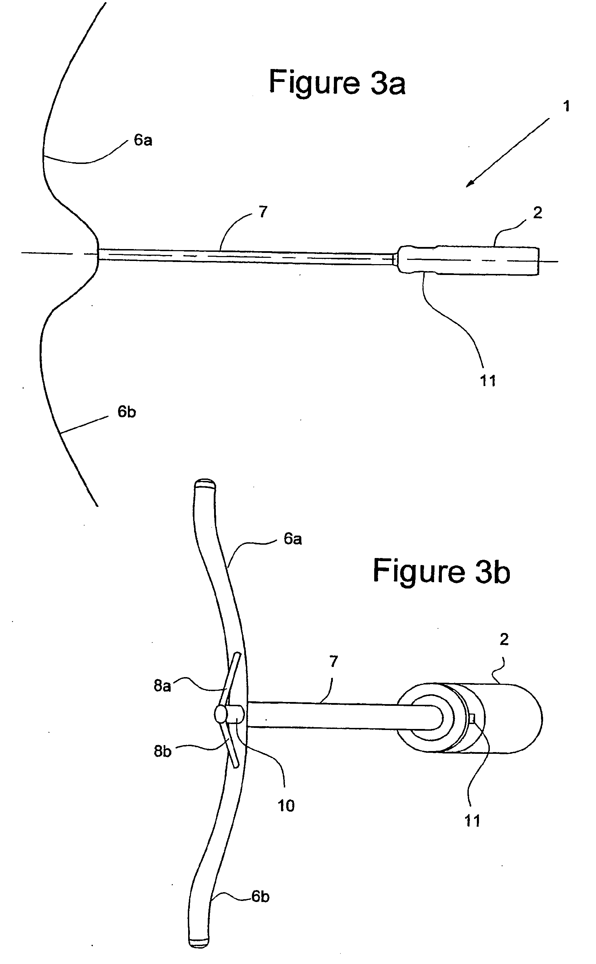

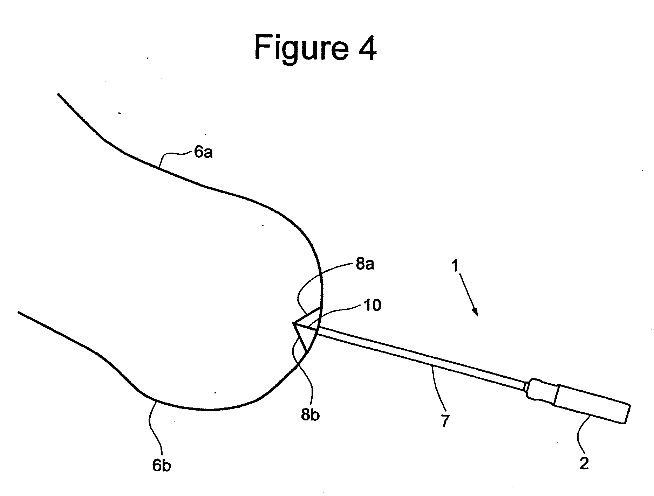

Selon le mode de réalisation représenté sur les figures, le dispositif de rappel comprend des éléments de rappel formés d'une lamelle souple à deux branches 8a, 8b disposées entre les lames 6a et 6b (voir figures 3b et 4). Les deux branches 8a, 8b forment ensemble un V dont chaque extrémité est reliée à l'une des deux lames 6a, 6b. Les extrémités des deux branches 8a et 8b de la lamelle souple prennent appui sur la face interne des lames 6a et 6b sur laquelle elles peuvent glisser, et leur mouvement est contrôlé par les bords des lames 6a et 6b qui sont relevés pour assurer le maintien en place des extrémités des branches 8a et 8b. Ainsi, les lames ont une section en U, les ailes du U étant constituées par le bord des lames. Les éléments de rappel comprennent également une tige filetée 9 s'étendant depuis la poignée 2 de l'écarteur 1 jusqu'aux lames souples 6a, 6b, et pénétrant au moins partiellement dans le tube rigide 3. L'extrémité distale 10 de la tige filetée 9 s'étend à l'extérieur du tube rigide 3, et vient en butée contre la lamelle, dans le V formé par les deux branches souples 8a et 8b.

Le dispositif de rappel comprend également des moyens permettant la commande des éléments de rappel, constitués par une molette 11 disposée sur la poignée 2 et agencée pour coopérer avec la tige filetée 9.

On décrit à présent le fonctionnement de l'écarteur représenté, lors d'une intervention laparoscopique de restauration aortique abdominale.

Avant que l'écarteur 1 ne soit introduit dans la cavité abdominale, la table d'opération doit être penchée de 30 degrés vers la droite afin que les anses intestinales du patient se trouvent dans la partie droite de son abdomen. L'écarteur 1 est alors introduit par l'intermédiaire du trocart à l'intérieur de la fosse iliaque. Les deux lames 6a, 6b sont alors dans leur première position, à l'intérieur de la gaine 7.

Les deux lames 6a, 6b sont poussées hors de la gaine 7 par un mouvement de simple retrait de la gaine, la gaine 7 venant coulisser sur le tube rigide 3. Les lames 6a, 6b peuvent ainsi se déployer automatiquement. Les figures 3a et 3b montrent l'écartement maximal théorique que pourraient atteindre les lames, prenant ainsi leur configuration en forme de S, si aucun filet ou aucune membrane n'était prévu entre elles. En pratique, lorsque les lames se déploient automatiquement, la présence du filet qui se déploie aussi automatiquement limite l'écartement entre elles.

Le chirurgien peut alors ajuster l'écartement entre les lames 6a, 6b en actionnant la molette 11, de sorte à placer les lames 6a, 6b dans leur deuxième position.

Si la molette 11 est tournée de façon à faire sortir la tige filetée 9 hors du tube 3, la tige viendra appuyer contre la lamelle, dans le V formé par les branches 8a, 8b et diminuera l'angle entre les deux branches 8a, 8b. Un tel mouvement a pour effet de rétrécir l'écartement entre les deux lames 6a, 6b en les rapprochant l'une dé l'autre (figure 4).

Si au contraire la molette 11 est tournée de façon à faire rentrer la tige filetée 9 à l'intérieur du tube 3, l'angle entre les deux branches 8a, 8b sera augmenté sous l'effet de l'élasticité de la lamelle, et les deux lames 6a, 6b s'éloigneront légèrement l'une de l'autre. Un tel mouvement est en pratique destiné à exercer une force permettant de maintenir un écartement stable entre les lames 6a, 6b. Cela peut être particulièrement utile lorsque les lames sont restées longtemps confinées dans la gaine, ce qui peut résulter, lors du déploiement automatique, en une configuration des lames 6a, 6b moins ferme.

L'ajustement réalisé par le chirurgien permet de déployer le filet en lui donnant la forme et la dimension adéquates pour envelopper et retenir les anses intestinales lors de l'intervention.

Lorsque l'écarteur 1 est correctement positionné dans l'abdomen, autour des anses intestinales, la table d'opération peut être remise en place, et le patient est alors placé en décubitus dorsal.

L'intervention sur l'aorte abdominale est pratiquée, puis l'écarteur 1 est retiré simplement en faisant rentrer les lames 6a, 6b à l'intérieur de la gaine 7, ce qui entraîne également le repliement automatique du filet sans autre manipulation.

Selon les matériaux utilisés pour sa fabrication, l'écarteur 1 selon l'invention peut être soit réutilisable, soit jetable.

Suivant une variante, le filet peut être jetable tandis que le manche et les lames de l'écarteur sont réutilisables.

## Revendications

1. Ecarteur laparoscopique (1) destiné à maintenir à distance un premier organe d'un deuxième organe lors d'une opération chirurgicale laparoscopique pratiquée sur ledit deuxième organe, ledit écarteur comprenant :
- une poignée de manipulation (2) apte à être fixée à une table d'opération;
- un tube creux rigide (3) comprenant une extrémité proximale (4) reliée à la poignée (2), et une extrémité distale (5) prolongée par au moins deux lames flexibles (6a, 6b) préformées, une extrémité de chaque lame (6a, 6b) étant fixée à l'extrémité distale (5) du tube (3), de telle sorte que les lames (6a, 6b), dans une première position, s'étendent sensiblement parallèlement l'une à l'autre ;
- un filet souple ou une membrane souple s'étendant entre les lames (6a, 6b), ledit filet ou ladite membrane étant de dimensions suffisantes pour permettre l'enveloppement au moins partiel dudit premier organe ;
- une gaine (7) sensiblement cylindrique agencée pour recevoir les lames (6a, 6b) dans leur première position, ladite gaine (7) étant montée de manière coulissante sur ledit tube (3) de sorte à permettre, lors de son coulissement vers le tube (3), le passage des lames (6a, 6b) de leur première position vers une deuxième position dans laquelle elles se déploient automatiquement de part et d'autre dudit tube (3) ;
ledit écarteur étant **caractérisé en ce qu'**il comprend un dispositif de rappel agencé pour ajuster et maintenir l'écartement entre les lames (6a, 6b), ledit dispositif comprenant :
- des éléments de rappel constitués d'au moins une lamelle souple à deux branches (8a, 8b) formant sensiblement un V, disposée entre lesdites lames (6a, 6b), chaque extrémité des branches du V étant reliée respectivement à chacune des deux lames (6a, 6b), au voisinage de l'extrémité distale (5) dudit tube (3), lesdits éléments de rappel comprenant en outre une tige filetée (9) s'étendant depuis la poignée (2) de l'écarteur (1) jusqu'au lames (6a, 6b), et pénétrant au moins partiellement dans le tube (3), ladite tige filetée (9) comprenant une extrémité distale (10) apte à s'étendre à l'extérieur du tube (3) lorsque les lames (6a, 6b) sont dans leur deuxième position, et venant en butée contre la au moins une lamelle dans le V formé par les deux branches (8a, 8b), et
- des moyens de commande des éléments de rappel agencés pour coopérer avec la tige filetée (9), lesdits moyens de commande étant disposés sur la poignée (2).

2. Ecarteur selon la revendication 1, **caractérisé en ce que** les moyens de commande sont constitués d'une molette (11) coopérant avec ladite tige filetée (9).

3. Ecarteur selon l'une des revendications précédentes, caractérisé l'extrémité distale de chaque branche de la lamelle en V coulisse dans une gouttière formée dans la face interne de la lame de l'écarteur

4. Ecarteur selon l'une des revendications précédentes, **caractérisé en ce que** les lames (6a, 6b) sont formées d'un métal souple, de type acier ou matière plastique injectée.

5. Ecarteur selon l'une quelconque des revendications précédentes, **caractérisé en ce que** le filet est formé de polypropylène.

6. Dispositif laparoscopique comprenant un écarteur (1) selon l'une quelconque des revendications 1 à 5 et un trocart endoscopique, **caractérisé en ce que** ledit écarteur (1) est placé à l'intérieur dudit trocart.

## Claims

1. A laparoscopic retractor (1) for keeping a first organ at a distance from a second organ during a laparoscopic surgical procedure performed on said second organ, said retractor comprising:
- a maneuvering handle (2) that can be fixed to an operating table;
- a hollow rigid tube (3) having a proximal end (4) connected to the handle (2) and a distal end (5) continued by at least two preformed flexible blades (6a, 6b), one end of each blade (6a, 6b) being fixed to the distal end (5) of the tube (3) in such a way that the blades (6a, 6b), in a first position, extend substantially parallel to each other;
- a flexible net or a flexible membrane extending between the blades (6a, 6b), said net or said membrane having sufficient dimensions to at least partially envelop said first organ;
- a substantially cylindrical sheath (7) designed to receive the blades (6a, 6b) in their first position, said sheath (7) being mounted so as to slide on said tube (3) in such a way that, during its sliding movement toward the tube (3), it allows the blades (6a, 6b) to change from their first position to a second position in which they automatically deploy on both sides of said tube (3);
said retractor being **characterized in that** it comprises a return device designed to adjust and maintain the spacing between the blades (6a, 6b), said device comprising:
- return elements composed of at least one flexible plate which has two branches (8a, 8b) forming substantially a V and which is arranged between said blades (6a, 6b), each end of the branches of the V being connected respectively to each of the two blades (6a, 6b), in proximity to the distal end (5) of said tube (3), said return elements further comprising a threaded rod (9) extending from the handle (2) of the retractor (1) to the blades (6a, 6b), and penetrating at least partially into the tube (3), said threaded rod (9) comprising a distal end (10) that can extend outside the tube (3) when the blades (6a, 6b) are in their second position, and that comes into abutment against the at least one plate in the V formed by the two branches (8a, 8b), and
- control means for controlling the return elements, designed to cooperate with the threaded rod (9), said control means being arranged on the handle (2).

2. The retractor as claimed in claim 1, **characterized in that** the control means are composed of a knurled wheel (11) that cooperates with said threaded rod (9).

3. The retractor as claimed in one of the preceding claims, **characterized in that** the distal end of each branch of the V-shaped plate slides in a channel formed in the inner face of the blade of the retractor.

4. The retractor as claimed in one of the preceding claims, **characterized in that** the blades (6a, 6b) are made of a flexible metal of the steel type or of an injected plastic material.

5. The retractor as claimed in any one of the preceding claims, **characterized in that** the net is made of polypropylene.

6. A laparoscopic device comprising a retractor (1) as claimed in any one of claims 1 through 5 and an endoscopic trocar, **characterized in that** said retractor (1) is placed inside said trocar.

## Patentansprüche

1. Laparoskopischer Abstandhalter (1), der dazu bestimmt ist, ein erstes Organ von einem zweiten Organ bei einer chirurgischen Operation, die an dem zweiten Organ durchgeführt wird, auf Abstand zu halten, wobei der Abstandhalter umfasst:
- einen Bediengriff (2), der an einem Operationstisch befestigbar ist,
- ein starres hohles Rohr (3), das ein proximales Ende (4) umfasst, das mit dem Griff (2) verbunden ist, und ein distales Ende (5), das von mindestens zwei vorgeformten flexiblen Stäben (6a, 6b) verlängert wird, wobei ein Ende jedes Stabs (6a, 6b) am distalen Ende (5) des Rohrs (3) derart befestigt ist, dass sich die Stäbe (6a, 6b) in einer ersten Stellung etwa parallel zueinander erstrecken,
- ein elastisches Netz oder eine elastische Membran, das/die sich zwischen den Stäben (6a, 6b) erstreckt, wobei das Netz oder die Membran ausreichend dimensioniert sind, um das zumindest teilweise Umhüllen des ersten Organs zu erlauben,
- eine etwa zylindrische Hülle (7), die ausgebildet ist, um die Stäbe (6a, 6b) in ihrer ersten Stellung aufzunehmen, wobei die Hülle (7) derart gleitend auf dem Rohr (3) montiert ist, dass bei ihrem Gleiten in Richtung des Rohrs (3) der Übergang der Stäbe (6a, 6b) aus ihrer ersten Stellung in eine zweite Stellung ermöglicht wird, in der sie sich automatisch auf der einen und der anderen Seite des Rohrs (3) entfalten,
wobei der Abstandhalter **dadurch gekennzeichnet ist, dass** er eine Rückstellvorrichtung umfasst, die ausgebildet ist, um den Abstand zwischen den Stäben (6a, 6b) zu justieren und aufrecht zu erhalten, wobei die Vorrichtung umfasst:
- Rückstellelemente, die aus mindestens einem elastischen Stäbchen mit zwei Armen (8a, 8b) besteht, die etwa ein V bilden, das zwischen den Stäben (6a, 6b) angeordnet ist, wobei jedes Ende der Arme des V jeweils mit jedem der zwei Stäbe (6a, 6b) in der Nähe des distalen Endes (5) des Rohrs (3) verbunden ist, wobei die Rückstellelemente ferner einen Gewindestab (9) umfassen, der sich ab dem Griff (2) des Abstandhalters (1) bis zu den Stäben (6a, 6b) erstreckt und zumindest teilweise in das Rohr (3) eindringt, wobei der Gewindestab (9) ein distales Ende (10) umfasst, das imstande ist, sich außerhalb des Rohrs (3) zu erstrecken, wenn sich die Stäbe (6a, 6b) in ihrer zweiten Stellung befinden, und gegen das mindestens eine Stäbchen im V, das von den zwei Armen (8a, 8b) gebildet wird, anschlägt, und
- Steuermittel der Rückstellelemente, die ausgebildet sind, um mit dem Gewindestab (9) zusammenzuarbeiten, wobei die Steuermittel auf dem Griff (2) angeordnet sind.

2. Abstandhalter nach Anspruch 1, **dadurch gekennzeichnet, dass** die Steuermittel aus einer Rändelmutter (11) bestehen, die mit dem Gewindestab (9) zusammenarbeitet.

3. Abstandhalter nach einem der vorangehenden Ansprüche, **dadurch gekennzeichnet, dass** das distale Ende jedes Arms des V-förmigen Stäbchens in einer Rinne gleitet, die in der Innenseite des Stabs des Abstandhalters eingearbeitet ist.

4. Abstandhalter nach einem der vorangehenden Ansprüche, **dadurch gekennzeichnet, dass** die Stäbe (6a, 6b) aus einem elastischen Metall vom Typ Stahl oder einem spritzgegossenen Kunststoffmaterial gebildet sind.

5. Abstandhalter nach einem der vorangehenden Ansprüche, **dadurch gekennzeichnet, dass** das Netz aus Polypropylen gebildet ist.

6. Laparoskopische Vorrichtung, die einen Abstandhalter (1) nach einem der Ansprüche 1 bis 5 umfasst und einen endoskopischen Trokar, **dadurch gekennzeichnet, dass** der Abstandhalter (1) im Trokar platziert ist.
